# EUROPEAN PATENT APPLICATION

(11) **EP 4 578 387 A1**
(43) Date of publication of application: **02.07.2025**
(21) Application number: 23857780.3
(22) Date of filing: 25.08.2023
(51) Int. Cl.: A61B 5/28, A61B 5/26, A61B 5/346, A61B 5/00, G16H 50/20

(54) **METHOD AND DEVICE FOR SIMULTANEOUSLY MEASURING SIX ELECTROCARDIOGRAM LEADS USING SMARTPHONE**

(30) Priority: 25.08.2022 KR 20220107069; 24.08.2023 KR 20230111545
(71) Applicant: Seoul National University Hospital, Seoul 03080 (KR)
(72) Inventor: KIM, Joonghee, Seongnam-si, Gyeonggi-do 13620 (KR)
(74) Representative: V.O.
(86) International application number: PCT/KR2023/012640
(87) International publication number: WO 2024/043748

(57) **Abstract**

Disclosed are a system, mobile terminal, and method of measuring biosignals according to one embodiment. The mobile terminal for measuring biosignals according to one embodiment measures biosignals of a subject, and comprises: one or more processors; memory; a housing including a screen area and a non-screen area; and one or more electrodes which are disposed in the non-screen area of the housing and sense the biosignals through contact with the subject, wherein the one or more processors output usage guidelines regarding the electrode contact of the subject to the screen area.

## Description

### Technical Field

The disclosed embodiments relate to a technique for simultaneously measuring up to six electrocardiogram leads using a smartphone, and more particularly, to a technique for simultaneously measuring six electrocardiogram leads with a combination of electrodes arranged at various locations of a smartphone.

### [Cross-reference to related application]

This application claims priority to Korean Provisional Patent Application No. 10-2022-0107069, filed on Aug. 25, 2022, and Korean Patent Application No.10-2023-0111545, filed on Aug. 24, 2023, the disclosures of which are incorporated by reference herein in their entirety.

### Background Art

With the advent of mobile and wearable technologies, it is possible to freely monitor biosignals without being restricted by time and space, beyond traditional medical institutions, thereby alleviating the spatial constraints of biosignal measurement.

However, existing wearable technologies rely on separate external devices for biosignal measurement. In other words, it is not possible to immediately measure biosignals in a mobile environment without necessary devices.

In addition, existing wearable technologies have improved the availability for the biosignal measurement, screens are limited in size or not mounted due to wear. This screen limitation makes it difficult to provide usage guidelines.

That is, the mobile and wearable technologies still require concise and intuitive usage guidelines. In particular, given that a subject is an individual who is not a healthcare professional, the manner of interaction with the measuring device must be easily explained.

### Detailed Description of the Invention

### Technical Problem

The disclosed embodiments are for simultaneously measuring six electrocardiogram leads using a smartphone.

### Solution to Problem

According to an embodiment, there is provided a mobile terminal for measuring biosignals of a subject, the mobile terminal including: one or more processors; memory; a housing including a screen area and a non-screen area; and one or more electrodes which are disposed in the non-screen area of the housing and sense the biosignals through contact with the subject, wherein the one or more processors output usage guidelines regarding the electrode contact of the subject to the screen area.

The one or more electrodes may include at least one of a first electrode, a second electrode, and a third electrode in a housing surrounding at least a part of the screen, wherein the first electrode may be disposed on a left short end of at least one of a front surface and a rear surface of the housing to be in contact with a left upper body of the subject, the second electrode may be disposed on a right short end of the at least one of the front surface and the rear surface of the housing to be in contact with a right upper body of the subject, and the third electrode may be disposed on the rear surface of the housing to be in contact with a lower body of the subject.

The one or more electrodes may include at least one of a first electrode, a second electrode, and a third electrode in a housing surrounding at least a part of the screen, wherein the first electrode may be disposed on a left end of each of a plurality of first side surfaces along a long axis with respect to a front surface of the housing to be in contact with a left upper body of the subject, the second electrode may be disposed on a right end of each of the plurality of first side surface along the long axis with respect the front surface of the housing to be in contact with a right upper body of the subject, and the third electrode may be disposed on a rear surface of the housing to be in contact with a lower body of the subject.

The one or more electrodes may include at least one of a first electrode, a second electrode, and a third electrode in a housing surrounding at least a part of the screen, wherein the first electrode may be disposed on a left edge of one of an upper end and a lower end of the housing to be in contact with a left upper body of the subject, the second electrode may be disposed in a right edge of the other of the upper end and the lower end of the housing to be in contact with a right upper body of the subject, and the third electrode may be disposed on one of a plurality of first side surfaces of the housing along a long axis to be in contact with an upper body of the subject.

The one or more electrodes may include at least one of a first electrode, a second electrode, and a third electrode in a housing surrounding at least a part of the screen, wherein the first electrode may be disposed on one of a plurality of first side surfaces of the housing along a long axis to be in contact with a hand of a subject gripping the housing, the second electrode may be disposed on one of the plurality of first side surfaces of the housing along the long axis and second side surfaces of the housing along a short axis to be in contact with another hand of the subject, and the third electrode may be disposed in the other of the plurality of second side surfaces of the housing along the short axis to be in contact with an upper body of the subject.

The one or more electrodes may include at least one of a first electrode, a second electrode, and a third electrode in a housing surrounding at least a part of the screen, wherein the first electrode may be disposed on each of a plurality of first side surfaces of the housing along a long axis to be in contact with a left upper body of the subject, the second electrode may be disposed on one of second side surfaces of the housing along a short axis to be in contact with a right upper body of the subject, and the third electrode may be disposed on the other of the second side surfaces of the housing along the short axis to be in contact with an upper body of the subject.

The one or more electrodes may include at least one of a first electrode, a second electrode, and a third electrode in a housing surrounding at least a part of the screen, wherein the first electrode may be disposed on one of a plurality of second side surfaces of the housing along a short axis to be in contact with a left upper body of the subject, the second electrode may be disposed on the other of the plurality of second side surfaces of the housing along the short axis to be in contact with a right upper body of the subject, and the third electrode may be disposed on at least one of the plurality of first side surfaces of the housing along a long axis to be in contact with an upper body of the subject.

The one or more processors may further determine whether the subject complies with the usage guidelines and output a notification signal based on the compliance with the usage guidelines.

The one or more processors may further output a physical examination result of the subject by using a pre-learned model when input information including biosignals of the subject is input.

The input information includes at least one of clinical information, baseline electrocardiogram information, and biosignals of the subject including 6-lead electrocardiogram (I, II, III, aVR, aVL, aVF), and the physical examination result of the subject may include at least one of a trend graph, a heart rhythm, a disease diagnosis, a heart function evaluation, and a physical condition evaluation.

The mobile terminal may include a smartphone.

According to an embodiment, there is provided a method of measuring biosignals performed by a mobile terminal, the method including: receiving, through one or more electrodes on the mobile terminal, biosignals of a subject in contact with the one or more electrodes; and outputting, on a screen of the mobile terminal, at least one of usage guidelines regarding electrode contact of the subject and a biosignal inspection result.

The method of measuring biosignals may include: determining whether the subject complies with the usage guidelines; and outputting a notification signal based on the compliance with the usage guidelines.

The outputting of the notification signal may include outputting a physical examination result of the subject by using a pre-learned model when the terminal receives the input information including the biosignals of the subject.

According to an embodiment, a system for measuring biosignals includes: a mobile terminal configured to measure the biosignals; a sensor configured to sense biosignals of a subject by using one or more electrodes in a housing of a device configured to measure the biosignals; and a server configured to acquire, learn, and analyze the biosignals of the subject, and provide an analysis result to the device configured to measure the biosignals.

The one or more electrodes may include at least one of a first electrode, a second electrode, and a third electrode in a housing surrounding at least a part of the screen, wherein the first electrode may be disposed on a left short end of at least one of a front surface and a rear surface of the housing to be in contact with a left upper body of the subject, the second electrode may be disposed on a right short end of at least one of the front surface and the rear surface of the housing to be in contact with a right upper body of the subject, and the third electrode may be disposed on the rear surface of the housing to be in contact with a lower body of the subject.

The one or more electrodes may include at least one of a first electrode, a second electrode, and a third electrode in the housing surrounding at least a part of the screen, wherein the first electrode may be disposed on a left end of each of a plurality of first side surfaces along a long axis with respect to a front surface of the housing to be in contact with a left upper body of the subject, the second electrode may be disposed on a right end of each of the plurality of first side surface along the long axis with respect the front surface of the housing to be in contact with a right upper body of the subject, and the third electrode may be disposed in a rear surface of the housing to be in contact with a lower body of the subject.

The one or more electrodes may include at least one of a first electrode, a second electrode, and a third electrode in the housing surrounding at least a part of the screen, wherein the first electrode may be disposed on a left edge of one of an upper end and a lower end of the housing to be in contact with a left upper body of the subject, the second electrode may be disposed in a right edge of the other of the upper end and the lower end of the housing to be in contact with a right upper body of the subject, and the third electrode may be disposed on one of a plurality of first side surfaces of the housing along a long axis to be in contact with an upper body of the subject.

The one or more electrodes may include at least one of a first electrode, a second electrode, and a third electrode in a housing surrounding at least a part of the screen, wherein the first electrode may be disposed on one of a plurality of first side surfaces of the housing along a long axis to be in contact with a hand of a subject gripping the housing, the second electrode may be disposed on one of the plurality of first side surfaces of the housing along the long axis and second side surfaces of the housing along a short axis to be in contact with another hand of the subject, and the third electrode may be disposed on the other of the plurality of second side surfaces of the housing along the short axis to be in contact with an upper body of the subject.

The one or more electrodes may include at least one of a first electrode, a second electrode, and a third electrode in a housing surrounding at least a part of the screen, wherein the first electrode may be disposed on each of a plurality of first side surfaces of the housing along a long axis to be in contact with a left upper body of the subject, the second electrode may be disposed on one of second side surfaces of the housing along a short axis to be in contact with a right upper body of the subject, and the third electrode may be disposed on the other of the second side surfaces of the housing along the short axis to be in contact with an upper body of the subject.

The one or more electrodes may include at least one of a first electrode, a second electrode, and a third electrode in a housing surrounding at least a part of the screen, wherein the first electrode may be disposed on one of a plurality of second side surfaces of the housing along a short axis to be in contact with a left upper body of the subject, the second electrode may be disposed on the other of the plurality of second side surfaces of the housing along the short axis to be in contact with a right upper body of the subject, and the third electrode may be disposed on at least one of the plurality of first side surfaces of the housing along a long axis to be in contact with an upper body of the subject.

The device for measuring biosignals may determine whether the subject complies with the usage guidelines, and further output a notification signal based on the compliance with the usage guidelines.

When the input information including the biosignals of the subject is input, the device for measuring biosignals may further output a physical examination result of the subject by using a pre-learned model.

The input information includes at least one of clinical information, baseline electrocardiogram information, and biosignals of the subject including 6-lead electrocardiogram (I, II, III, aVR, aVL, aVF), and the physical examination result of the subject may include at least one of a trend graph, a heart rhythm, a disease diagnosis, a heart function evaluation, and a physical condition evaluation.

### Effects of the Invention

The disclosed embodiments may simultaneously measure up to six electrocardiogram leads of a subject at various locations when the subject grips a mobile terminal for measuring biosignals with electrodes in a housing.

The disclosed embodiments may allow the subject to grip the electrodes provided at locations that do not obscure the screen through strategic electrode placement, thereby providing usage guidelines on the screen during use.

### Brief Description of Drawings

FIG. 1 is a block diagram of a system for measuring biosignals according to an embodiment.
FIG. 2 is a block diagram of a mobile terminal according to an embodiment.
FIG. 3 is an example diagram illustrating a first arrangement of one or more electrodes provided in a mobile terminal according to an embodiment.
FIG. 4 is an example diagram illustrating a second arrangement of one or more electrodes provided in a mobile terminal according to an embodiment.
FIG. 5 is an example diagram illustrating a third arrangement of one or more electrodes provided in a mobile terminal according to an embodiment.
FIG. 6 is an example diagram illustrating a fourth arrangement of one or more electrodes provided in a mobile terminal according to an embodiment.
FIG. 7 is an example diagram illustrating a fifth arrangement of one or more electrodes provided in a mobile terminal according to an embodiment.
FIG. 8 is an example diagram illustrating a sixth arrangement of one or more electrodes provided in a mobile terminal according to an embodiment.
FIG. 9 is an example diagram of an architecture of a pre-learned model used for a mobile terminal according to an embodiment.
FIG. 10 is an example diagram illustrating a software operation method of a device for measuring biosignals according to an embodiment.
FIG. 11 is a flowchart of a method of measuring biosignals according to an embodiment.
FIG. 12 is a flowchart of a method of measuring biosignals according to an additional embodiment.

### Best Mode for Carry out the Invention

Hereinafter, embodiments are described in detail with reference to the drawings. The following detailed description is provided to facilitate a comprehensive understanding of sensors described herein. However, this is merely an example. The present invention is not limited thereto.

In describing the embodiments, detailed descriptions of well-known technologies related to the present invention will be omitted when it is determined that the detailed descriptions may unnecessarily obscure the subject matter of the embodiments. In addition, numbers (e.g., first, second, and the like) used in the description of the embodiments are merely identifiers for distinguishing one component from another component.

In the description of the embodiments, when an element is referred to as being "on", "above", "on top of", "under", "below", or "on the bottom of" another element, this includes not only a case where the element is in contact with the other element but also a case where there is another element between the two elements.

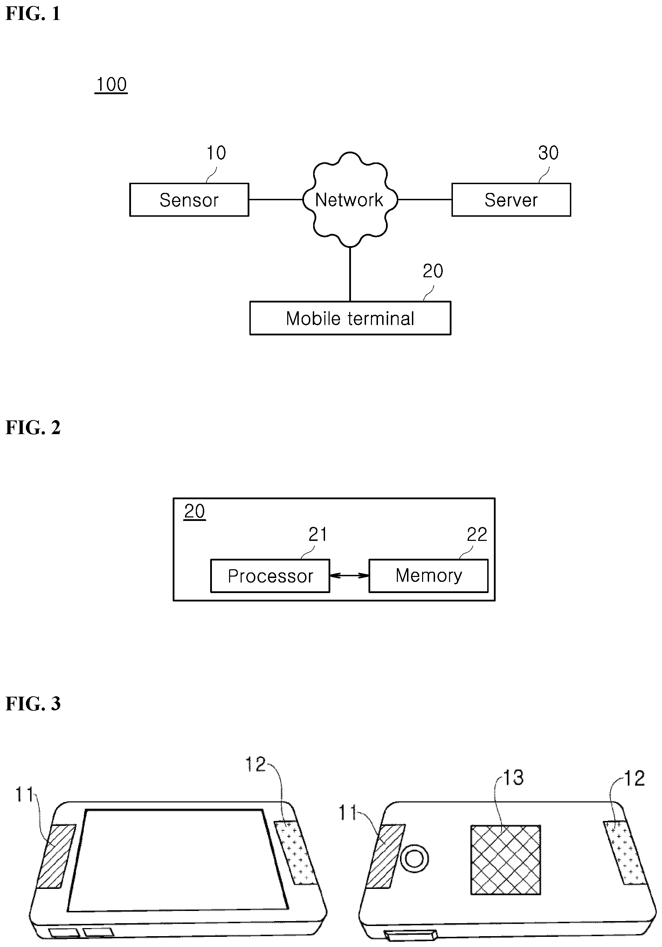

FIG. 1 is a block diagram of a system 100 for measuring biosignals according to an embodiment.

Referring to FIG. 1, the system 100 for measuring biosignals includes a sensor 10, a mobile terminal 20, and a server 30.

The system 100 for measuring biosignals, according to an embodiment, measures biosignals by using the sensor 10 disposed on the mobile terminal 20, sends the measured biosignals to the server 30 for analysis, and then provides a physical condition result to a subject.

The sensor 10 may sense the biosignals of the subject by using one or more electrodes on the housing of the mobile terminal 20. For example, one or more sensors 10 may sense an electrocardiogram (ECG) of the subject in contact with the sensors 10 when the subject grips the mobile terminal 20.

The mobile terminal 20 may collect the biosignals sensed by the sensor 10, evaluate the quality of the collected biosignals, and output usage guidelines for measuring biosignals on the screen to provide the same to the subject.

The mobile terminal 20 may include, for example, at least one of a smartphone, a tablet personal computer (PC), a mobile phone, a video phone, an e-book reader, a desktop PC, a personal digital assistant (PDA), a portable multimedia player (PMP), an MP3 player, a mobile medical device, a wearable device, an electronic garment, an electronic bracelet, an electronic necklace, an appcessory, an electronic tattoo, a smart mirror, and a smart watch.

The server 30 may form a communication channel with the mobile terminal 20 via a network and receive biosignals from the mobile terminal 20. The server 30 may learn, store, perform analysis on the biosignals, compare the analysis result with a pre-stored condition or result, and provide a physical examination result of the subject.

Although the sensor 10 is described as a separate component from the mobile terminal 20 in FIG. 1, the sensor 10 may be an internal component embedded in the mobile terminal 20, unlike the illustrated example.

FIG. 2 is a block diagram of the mobile terminal 20 according to an embodiment.

Referring to FIG. 2, the mobile terminal 20 according to an embodiment includes a processor 21 and memory 22, a housing (not shown), and one or more electrodes.

The processor 21 may perform data processing related to at least one of control and communication of one or more components for measuring biometric information.

Specifically, the processor 21 may obtain, preprocess, analyze, store, and output the biosignals through the one or more electrodes on the mobile terminal 20.

For example, the processor 21 may obtain a 1-lead ECG through biometric information measured from the upper body of the subject. The processor 21 may generate the 1-lead ECG by obtaining biosignals of both hands of the subject in contact with the one or more electrodes (e.g., a first electrode 11 and a second electrode 12 to be described below) on the housing (not shown) of the mobile terminal 20.

In another example, the processor 21 may obtain a 6-lead ECG through biometric information measured from the upper and lower bodies of the subject. The processor 21 may generate the 6-lead ECG through biosignals of the abdomen or the flanks, and both hands of the subject in contact with the one or more electrodes (e.g., a first electrode 11, a second electrode 12, and a third electrode 13 to be described below) on the housing (not shown) of the mobile terminal 20.

That is, the processor 21 may measure the biosignals of the subject by using one or more electrodes in contact with the subject when the subject grips the mobile terminal 20. In particular, the processor 21 may determine the type of ECG to be generated based on the number of electrodes held to measure biosignals.

On the other hand, the processor 21 may measure the biosignals by physically and/or electrostatically sensing the electrical characteristics and/or the pressure applied by the subject through the one or more electrodes.

The processor 21 may provide the subject with a correct gripping method for precise biosignal measurement. The processor 21 may provide the subject with the usage guidelines on the screen in the mobile terminal 20 while measuring the biosignals.

The processor 21 may output information on the correct usage guidelines (e.g., at least one of a gripping position, an intensity, a strength, a pressure, a contact failure, a measurement time, and a measurement posture) to the screen in the mobile terminal 20.

The processor 21 may determine whether the subject complies with the usage guidelines and output a notification signal based on the compliance with the usage guidelines. When the processor 21 determines that the subject does not comply with the usage guidelines, the processor 21 may output at least one of visual, auditory, and tactile notification signals.

The processor 21 may input the input information generated based on the biosignals obtained through the one or more electrodes on the mobile terminal 20 into a pre-learned model to analyze, generate, store, or output the physical examination result of the subject.

The input information may be generated based on at least one of biosignals, baseline ECG information, and clinical information obtained through the one or more electrodes on the mobile terminal 20.

Specifically, the processor 21 may generate a first biometric embedding vector based on biosignals obtained in real-time through the one or more electrodes, i.e., the 1-lead or 2-lead ECG or the ECG with more leads made through or a linear combination of 2-lead (e.g., making up all or some of leads III, aVR, aVL, aVF through a linear combination of leads I and II). That is, the processor 21 may generate a single-channel or multi-channel one-dimensional numeric array as the first biometric embedding vector.

These biosignals may be obtained through multiple measurements using one or more electrodes or may be obtained by changing the measurement position (e.g., contacting the anterior thorax to obtain V1, V2, V3, V4, V5, V6) to generate and analyze biosignals composed of all or some of the ECG leads (I, II, III, aVR, aVL, aVF, V1, V2,V3, V4,V5, V6) using the processor 21.

The processor 21 may generate the input information based on the obtained baseline ECG information. The baseline ECG information refers to an ECG status representing a normal physical condition of a patient. The baseline ECG information may be obtained prospectively through periodic or random activated notifications or may be obtained retrospectively by being pre-stored in the mobile terminal 20.

The processor 21 may generate a second biometric embedding vector based on the obtained baseline ECG information or the preprocessed baseline ECG. The second biometric embedding vector may be generated in a similar manner to the first biometric embedding vector.

The processor 21 may standardize, normalize, or encode, as clinical information, at least one of an age, a gender, a height, a weight, an underlying condition (e.g., hypertension, diabetes, hyperlipidemia, cerebral infarction, myocardial infarction, or peripheral artery atresia), and a symptom (e.g., chest pain, dyspnea, syncope, or dizziness), to generate a third biometric embedding vector.

The clinical information may include information directly input from the subject, directly measured through equipment (e.g., sensors) provided in the mobile terminal 20, or transmitted from external equipment or a cloud service through communication.

The processor 21 may concatenate at least some of the first to third biometric embedding vectors to generate input information for input to the pre-learned model.

In this case, the processor 21 may use only the first to third biometric embedding vectors that satisfy the condition as the input information for good-quality prediction. For example, the processor 21 may use only biometric embedding vectors with a preset upload time, size, and/or capacity as the input information. For another example, the processor 21 may exclude, as the input information, newly acquired ECG information, baseline ECG information, and/or clinical information if they are older than a preset date.

The processor 21 may input the generated input information into at least one of the plurality of pre-learned models to analyze, generate, store, or output the physical examination result of the subject.

Specifically, the processor 21 may select the pre-learned model to be used for predicting the physical examination result based on the number of information types included in the input information. Alternatively, the processor 21 may select the pre-learned model to be used for predicting the physical examination result based on the configuration of electrodes (e.g., at least one of the type and the number of electrodes) in contact with the subject to measure biosignals.

The pre-learned model may be designed to be built outside the mobile terminal 20 or lightweight built inside the mobile terminal 20 to perform some or all of the tasks simultaneously.

The processor 21 may output, as the physical examination result of the subject, at least one of a trend graph, a heart rhythm, a disease diagnosis, a heart function evaluation, and a physical condition evaluation on the screen.

The trend graph is a graph indicating a relationship between a time and a numerical value obtained by evaluating a variable related to health. The trend graph may refer to a graph showing a change, a pattern, and a trend of the physical condition over a sequential period. That is, the processor 21 may visually provide at least one of a trend, a change, a pattern, and an outlier of the physical condition over time.

The cardiac rhythm may include at least one of atrial fibrillation, atrial flutter, atrial tachycardia, wandering atrial pacemaker, multifocal atrial tachycardia, ventricular tachycardia, ventricular fibrillation, idioventricular rhythm, paroxysmal supraventricular tachycardia, pre-excitation, junctional rhythm, AV block (stage 1, stage 2, stage 3), escape rhythm, pacemaker rhythm, and pacemaker malfunction.

The disease diagnosis may include whether at least one of cardiac arrest, respiratory failure, myocardial infarction, myocardium injury, and pulmonary edema has developed. The cardiac function assessment may include at least one of left ventricular failure, right ventricular failure, cardiac structural changes (e.g., left ventricular hypertrophy (LVH), right ventricular hypertrophy (RVH), left atrial enlargement (LAE), and right atrial enlargement (RAE)), and valvular dysfunction. The physical condition evaluation may include at least one of health age, life expectancy, risk of developing cardiovascular disease, exercise capacity, and preoperative risk.

The memory 22 stores one or more instructions executed by the processor 21.

The memory 22 may store various data used by the processor 21. For example, the memory 22 may include software (e.g., input data or output data for a program executed by the processor 21 and/or instructions related to the program).

The memory 22 may include volatile memory or nonvolatile memory.

FIG. 3 is an example diagram illustrating a first arrangement of one or more electrodes provided in the mobile terminal 20 according to an embodiment.

FIG. 3 illustrates a procedure for measuring biosignals of both hands and the lower body by a 3-electrode method.

Referring to FIG. 3, the mobile terminal 20 according to an embodiment includes one or more electrodes of the first electrode 11, the second electrode 12, and the third electrode 13.

The one or more electrodes are implemented inside or outside the mobile terminal 20 to receive the biosignals of the subject in contact with the mobile terminal 20.

The one or more electrodes may be disposed on the edge or the rear surface of the housing for measuring the biosignals, to prevent the screen from being covered by the subject in contact with the mobile terminal 20 through gripping.

As an example, the first electrode 11 may be disposed on a left short end of at least one of a front surface and a rear surface of the housing to be in direct or indirect contact with a left upper body (e.g., a left hand) of the subject.

As an example, the second electrode 12 may be disposed on a right short end of at least one of a front surface and a rear surface of the housing to be in direct or indirect contact with a right upper body (e.g., a right hand) of the subject.

As an example, the third electrode 13 may be disposed on the rear surface of the housing to be in direct or indirect contact with the lower body (e.g., thigh or knee) of the subject.

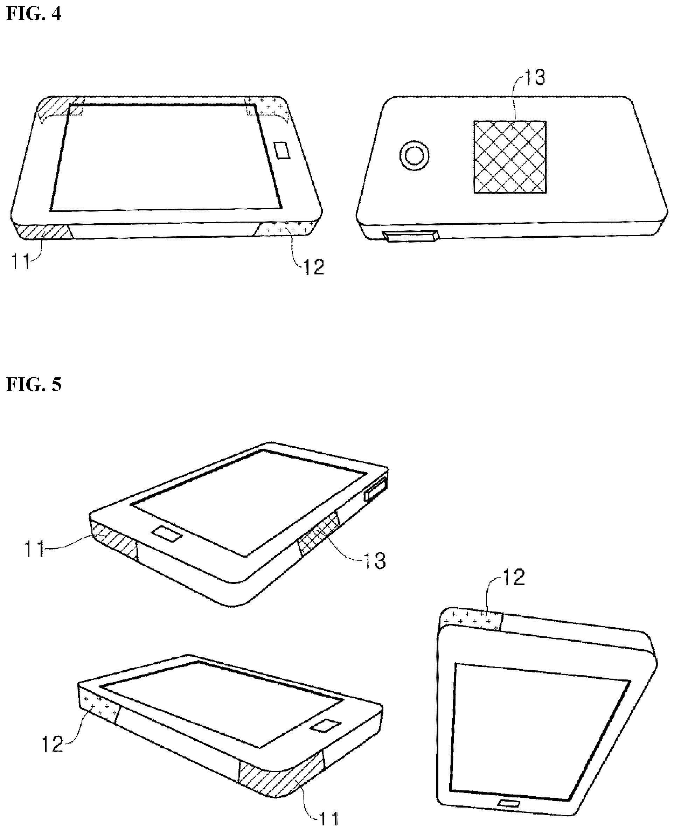

FIG. 4 is an example diagram illustrating a second arrangement of one or more electrodes provided in the mobile terminal 20 according to an embodiment.

FIG. 4 illustrates a procedure for measuring biosignals of both hands and the lower body by a 3-electrode method.

For convenience of description, redundant description of FIG. 3 is omitted in FIG. 4.

Referring to FIG. 4, the mobile terminal 20 according to an embodiment includes at least one of the first electrode 11, the second electrode 12, and the third electrode 13 in the housing surrounding at least a part of the screen.

As an example, the first electrode 11 may be disposed on a left end of each of a plurality of first side surfaces along the long axis with respect to the front surface of the housing to be in direct or indirect contact with the left upper body (e.g., the left hand) of the subject.

As an example, the second electrode 12 may be disposed on a right end of each of the plurality of first side surfaces along the long axis with respect to the front surface of the housing to be in direct or indirect contact with the right upper body (e.g., the right hand) of the subject.

As an example, the third electrode 13 may be disposed on the rear surface of the housing to be in direct or indirect contact with the lower body (e.g., the thigh or the knee) of the subject.

FIG. 5 is an example diagram illustrating a third arrangement of one or more electrodes provided in the mobile terminal 20 according to an embodiment.

FIG. 5 illustrates a procedure for measuring the biosignals of both hands and the upper body by the 3-electrode method.

For convenience of description, redundant descriptions of FIG. 3 and FIG. 4 are omitted in FIG. 5.

Referring to FIG. 5, the mobile terminal 20 according to an embodiment includes at least one of a first electrode 11, a second electrode 12, and a third electrode 13 in the housing surrounding at least a part of the screen.

As an example, the first electrode 11 may be disposed on a left edge of one of the upper end and the lower end of the housing to be in direct or indirect contact with the left upper body (e.g., the left hand) of the subject.

As an example, the second electrode 12 may be disposed on a right edge of the other of the upper end and the lower end of the housing to be in direct or indirect contact with the right upper body (e.g., the right hand) of the subject.

As an example, the third electrode 13 may be disposed on one of the plurality of first side surfaces of the housing along the long axis to be in direct or indirect contact with the upper body (e.g., chest, abdomen, pelvis, or flank) of the subject.

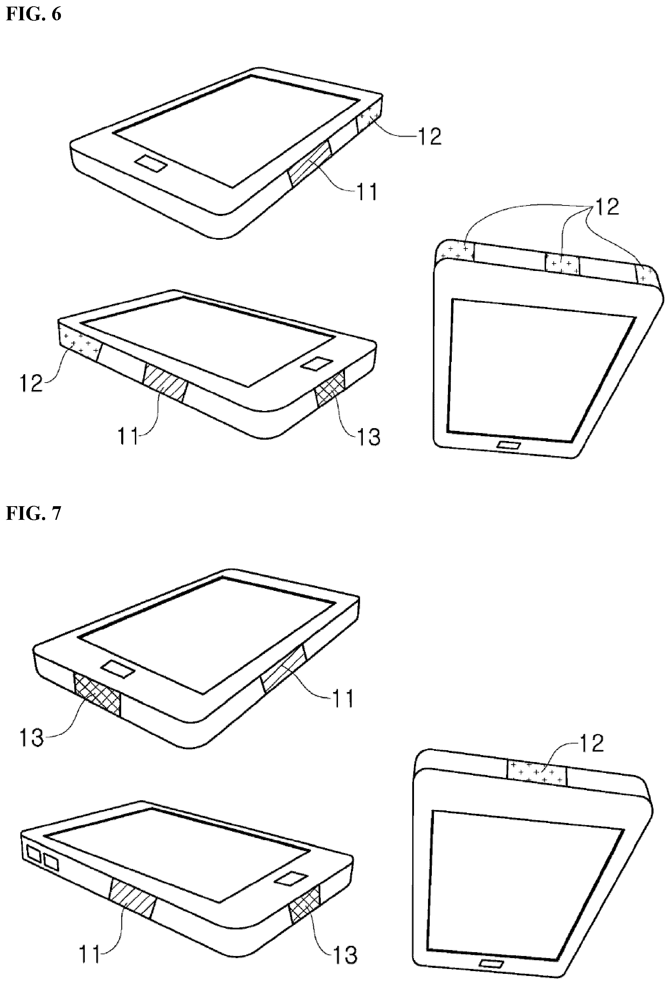

FIG. 6 is an example diagram illustrating a fourth arrangement of one or more electrodes provided in the mobile terminal 20 according to an embodiment.

FIG. 6 illustrates a procedure for measuring the biosignals of both hands and the upper body by the 3-electrode method.

For convenience of description, redundant descriptions of FIGS. 3 to 5 are omitted in FIG. 6.

Referring to FIG. 6, the mobile terminal 20 according to an embodiment includes at least one of the first electrode 11, the second electrode 12, and the third electrode 13 in the housing surrounding at least a part of the screen.

As an example, the first electrode 11 may be disposed on one of the plurality of first side surfaces of the housing along the long axis to be in direct or indirect contact with one hand of the subject gripping the housing.

As an example, the second electrode 12 may be disposed on one of the plurality of first side surfaces of the housing along the long axis and the second side surface of the housing along the short axis to be in direct or indirect contact with the other hand of the subject.

As an example, the third electrode 13 may be disposed on one of the plurality of second side surfaces of the housing along the short axis, other than the second electrode 12, to be in direct or indirect contact with an upper body of the subject.

FIG. 7 is an example diagram illustrating a fifth arrangement of one or more electrodes provided in the mobile terminal 20 according to an embodiment.

In FIG. 7, a process for measuring biosignals of both hands and the upper body by the 3-electrode method with the screen set in a vertical direction with reference to the subject is described.

For convenience of description, redundant descriptions of FIGS. 3 to 6 are omitted in FIG. 7.

Referring to FIG. 7, the mobile terminal 20 according to an embodiment includes at least one of the first electrode 11, the second electrode 12, and the third electrode 13 in the housing surrounding at least a part of the screen.

As an example, the first electrode 11 may be disposed on each of the plurality of first side surfaces of the housing along the long axis to be in direct or indirect contact with the left upper body (e.g., the left hand) of the subject.

As an example, the second electrode 12 may be disposed on one of the second side surfaces of the housing along the short axis to be in direct or indirect contact with the right upper body (e.g., the right hand) of the subject.

As an example, the third electrode 13 may be disposed on a side surface other than the second electrode 12 among the second side surfaces of the housing along the short axis, to be in direct or indirect contact with the upper body (e.g., the chest, the abdomen, the pelvis, or the flanks) of the subject.

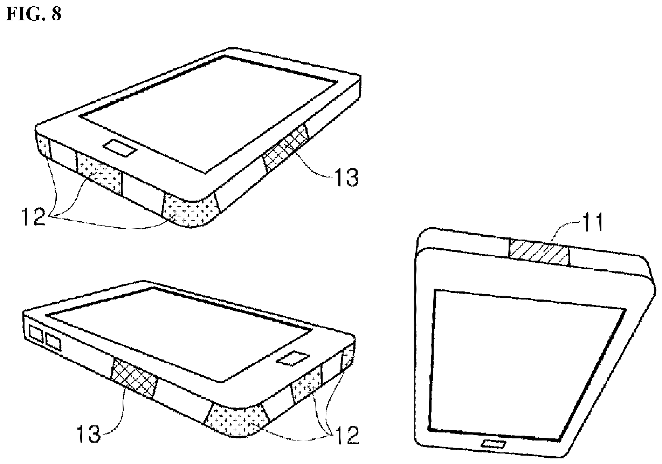

FIG. 8 is an example diagram illustrating a sixth arrangement of one or more electrodes provided in the mobile terminal 20 according to an embodiment.

In FIG. 8, a process for measuring the biosignals of both hands and the upper body horizontally by the 3-electrode method with the screen in a horizontal direction with reference to the subject is described.

For convenience of description, redundant descriptions of FIGS. 3 to 7 are omitted in FIG. 8.

Referring to FIG. 8, the mobile terminal 20 according to an embodiment includes at least one of the first electrode 11, the second electrode 12, and the third electrode 13 in the housing surrounding at least a part of the screen.

As an example, the first electrode 11 may be disposed on one of the plurality of second side surfaces of the housing along the short axis to be in direct or indirect contact with the left upper body (e.g., the left hand) of the subject.

As an example, the second electrode 12 may be disposed on a side surface other than the first electrode 11 among the plurality of second side surfaces of the housing along the short axis, as well as on edges located on both sides of the other surface, to be in direct or indirect contact with the right upper body (e.g., the right hand) of the subject.

As an example, the third electrode 13 may be disposed on at least one of the plurality of first side surfaces of the housing along the long axis to be in direct or indirect contact with the upper body (e.g., the abdomen or the side) of the subject.

FIGS. 2 to 8 show that the first electrode 11 to the third electrode 13 are in fixed locations for convenience of description, but this is an example. The first electrode 11 to the third electrode 13 may be rearranged in a predefined area by changing the arrangement order.

Although FIGS. 5 to 8 are described with reference to a particular hand (left hand) gripping the mobile device, this is an example. The arrangement of the electrodes may be rearranged with reference to the other hand gripping.

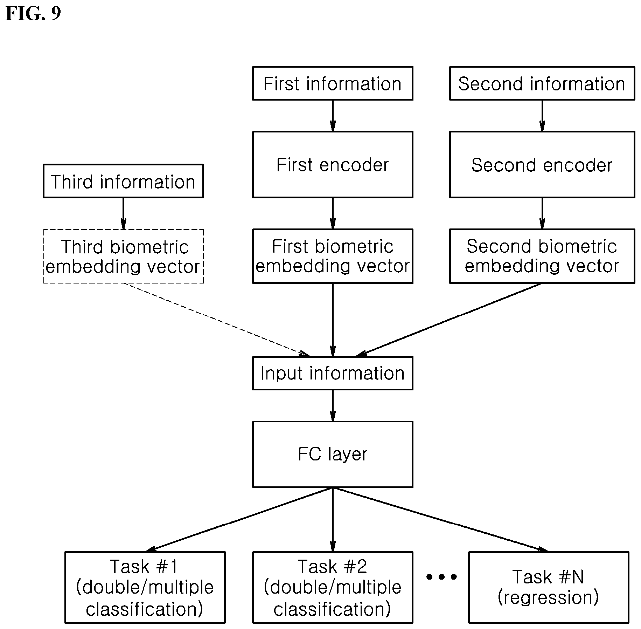

FIG. 9 is an example diagram of an architecture of a pre-learned model used for a mobile terminal according to an embodiment.

Referring to FIG. 9, the pre-learned model includes a first encoder, a second encoder, and a fully connected layer (FC Layer).

The pre-learned model may receive, as first information, the ECG signal obtained in real time through the one or more electrodes on the mobile terminal 20.

The pre-learned model may generate the first biometric embedding vector based on the first information corresponding to the ECG signal through the first encoder.

The first encoder may include at least one of a convolutional neural network (CNN), a recurrent neural network (RNN), a multi-layer perceptron (MLP), and a transformer.

The pre-learned model may generate, through the second encoder, the second biometric embedding vector based on the second information corresponding to the clinical information of the subject.

The second encoder may include at least one of the MLP and the transformer.

The pre-learned model may further generate the third biometric embedding vector based on the obtained baseline ECG information or the preprocessed baseline ECG to improve prediction accuracy.

The pre-learned model may then concatenate at least two or more of the first to third biometric embedding vectors to generate input information for input to the pre-learning model.

Note that the terms "first", "second", and the like are used here only for the purpose of distinguishing one component from another component and are not limited to the terms.

In this case, the pre-learned model may simultaneously or separately perform various tasks by using the FC layer included in the model. For example, the task may perform double or multiple classification or regression for predicting consecutive numerical values, or the like.

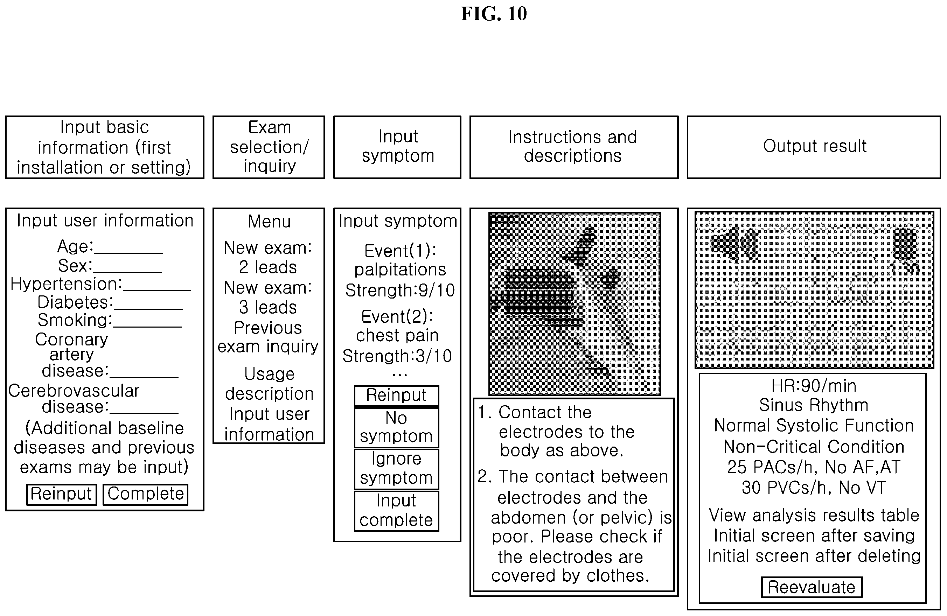

FIG. 10 is an example diagram sequentially illustrating a tutorial of the mobile terminal 20 according to an embodiment.

Referring to FIG. 10, the mobile terminal 20 according to an embodiment provides input/output experiences and a tutorial to the subject through the screen.

The mobile terminal 20 according to an embodiment may provide an input field for obtaining clinical information including an age, a gender, a medical history (e.g., hypertension, diabetes, coronary artery disease, or cerebrovascular disease), and a smoking status on the screen.

Subsequently, the mobile terminal 20 according to an embodiment may display an option for selecting a biosignal measurement method from the subject on the screen. For example, the mobile terminal 20 according to an embodiment may display an option for selecting a measurement method classified according to the number of used electrodes (e.g., two or three) on the screen.

Subsequently, the mobile terminal 20 according to an embodiment may receive at least one of the baseline ECG information and the clinical information on which the input information is based. The mobile terminal 20 according to an embodiment may display a subject interface for retrieving or inputting at least one of the baseline ECG information and the clinical information on the screen.

Subsequently, the mobile terminal 20 according to an embodiment may activate connection with the one or more electrodes to receive biosignals of the subject in contact with the one or more electrodes.

At the same time, the mobile terminal 20 according to an embodiment may display information describing usage guidelines for providing a measurement method on the screen. The mobile terminal 20 may display at least one of a still image and a moving image for describing the usage guidelines on the screen. In this case, the mobile terminal 20 according to an embodiment may provide the usage guidelines including a correct gripping method when the usage guidelines of the determined measurement method do not match the contact method of the subject with the electrodes.

Subsequently, the mobile terminal 20 according to an embodiment may output, on the screen, the biosignal information measured in real time by gripping the subject. In this case, the mobile terminal 20 according to an embodiment may further output a notification signal for notifying output of the biosignal information of the subject. Alternatively, the mobile terminal 20 according to an embodiment may output, on the screen, the display time of the biosignal information currently displayed on the screen.

Subsequently, the mobile terminal 20 according to an embodiment may output a physical examination result of the subject corresponding to the input information by using the pre-learned model.

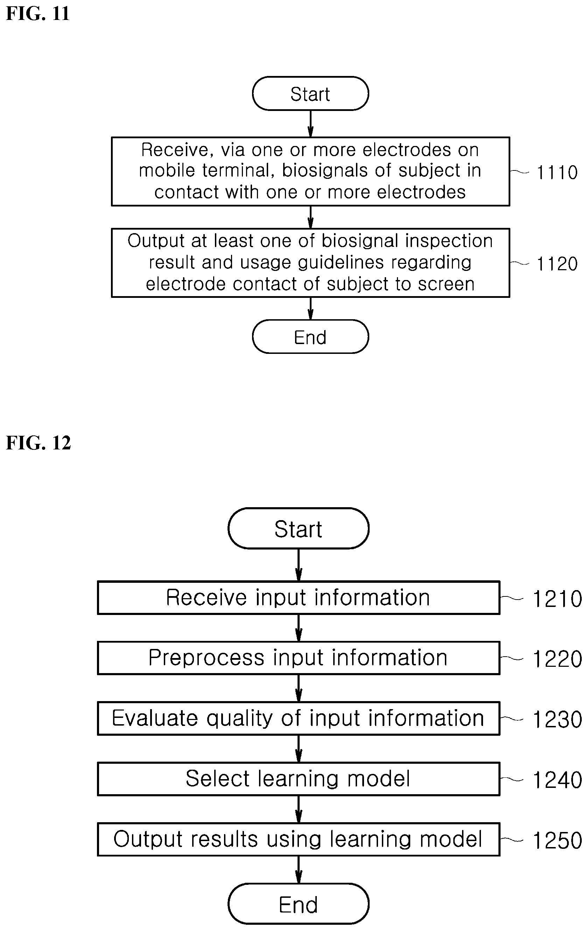

FIG. 11 is a flowchart of a method of measuring biosignals according to an embodiment.

Referring to FIG. 11, the method of measuring biosignals according to an embodiment may be performed by the mobile terminal 20 of FIG. 2.

First, the mobile terminal 20 receives biosignals of the subject in contact with one or more electrodes on the mobile terminal 20 through the one or more electrodes (1110).

Subsequently, the mobile terminal 20 outputs at least one of the usage guidelines regarding the electrode contact of the subject and the biosignal inspection result to the screen of the mobile terminal (1120).

FIG. 12 is a flowchart of a method of measuring biosignals according to an additional embodiment.

Referring to FIG. 12, the method of measuring biosignals according to an embodiment may be performed by the mobile terminal 20 of FIG. 2.

First, the mobile terminal 20 may receive input information (1210). The input information may refer to raw data on which the first to third biometric embedding vectors are based, which is directly input from the user or measured by the mobile terminal 20 or other external devices.

The mobile terminal 20 preprocesses the input information. The mobile terminal 20 may preprocess the input information to satisfy a predefined format (1220).

For example, the mobile terminal 20 may perform filtering for noise reduction and/or highlighting of a specific section in response to consecutive ECG signals. Alternatively, the mobile terminal 20 may perform encoding or scaling according to the data type of the obtained clinical information of the subject.

The mobile terminal 20 may evaluate the quality of the input information (1230). The mobile terminal 20 may determine whether the input information meets a predefined condition to select desirable input information.

The mobile terminal 20 selectively filters the input information based on whether the input information meets the predefined condition. Specifically, if the input information meets the predefined condition, the input information may be input to the model and analyzed. If the input information does not meet the predefined condition, the input information may be discarded.

The mobile terminal 20 may select one or more learning models for analyzing the selected input information (1240). Specifically, the mobile terminal 20 may select the learning models to be used for analysis based on at least one of the number of used electrodes and the type of electrodes.

The learning models may be lightweight built in the mobile terminal 20 or may be built in an external server. Specifically, the learning models embedded in the mobile terminal 20 may be structurally designed to reduce its function, size, parameter number, computation amount, and the like through a weight reduction operation (e.g., pruning or quantization).

Subsequently, the mobile terminal 20 may output at least one of the usage guidelines regarding the electrode contact and the biosignal inspection result by using the learning models (1250).

Although the method is described as being divided into a plurality of steps in the illustrated flowchart, at least some of the steps may be performed in a reverse order, may be combined with other steps and performed together, may be omitted, may be divided into detailed steps, or may be performed by adding one or more steps that are not illustrated.

An embodiment of the present invention may include a program for performing the methods described herein on a computer, and a computer-readable recording medium including the program. The computer-readable recording medium may include program instructions, local data files, local data structures, and the like, alone or in combination. The medium may be specially designed and constructed for the present invention or may be commonly used in the field of computer software. Examples of the computer-readable recording medium include magnetic media, such as a hard disk, a floppy disk, and a magnetic tape, optical recording media, such as CD-ROM and DVD, and hardware devices specifically configured to store and perform program instructions, such as ROM, RAM, flash memory, and the like. Examples of the program may include machine language codes, such as those made by a compiler, as well as high-level language codes executable by a computer using an interpreter or the like.

While representative embodiments of the present invention have been described in detail above, those skilled in the art may understand that various modifications can be made to the above-described embodiments without departing from the scope of the present invention. Therefore, the scope of the present invention should not be limited to the described embodiments, but should be defined by the following claims and their equivalents.

The terms described below are terms defined in consideration of functions in the present invention, which may vary depending on a subject, and an operator's intention or practice. Therefore, the terms should be defined based on the contents throughout the specification. The terms used herein is only for describing embodiments and not for limiting the same. The singular expressions include plural expressions unless the context clearly dictates otherwise. In the present description, expressions such as "comprising" or "including" are intended to indicate certain components, numbers, steps, operations, elements, parts or combinations thereof, and should not be interpreted to exclude the presence or possibility of one or more other components, numbers, steps, operations, elements, parts, or combinations thereof other than those described above.

Furthermore, embodiments described herein may have aspects that are entirely in hardware, partly in hardware and partly in software, or entirely in software. A unit herein refers to hardware, a combination of hardware and software, or a computer-related entity, such as software.

### Industrial Availability

A system, a mobile terminal, and a method of measuring biosignals according to an embodiment measure biosignals through electrodes strategically disposed in the mobile terminal, and analyze the measured biosignals, which are available to the digital medical industry.

## Claims

1. A mobile terminal for measuring biosignals of a subject, the mobile terminal comprising:
one or more processors;
memory;
a housing comprising a screen area and a non-screen area; and
one or more electrodes disposed in the non-screen area of the housing and sensing the biosignals through contact with the subject, wherein the one or more processors output usage guidelines regarding the electrode contact of the subject to the screen area.

2. The mobile terminal of claim 1, wherein the one or more electrodes comprise at least one of a first electrode, a second electrode, and a third electrode in the housing surrounding at least a part of the screen, wherein the first electrode is disposed on a left short end of at least one of a front surface and a rear surface of the housing to be in contact with a left upper body of the subject, the second electrode is disposed on a right short end of at least one of the front surface and the rear surface of the housing to be in contact with a right upper body of the subject, and the third electrode is disposed on the rear surface of the housing to be in contact with a lower body of the subject.

3. The mobile terminal of claim 1, wherein the one or more electrodes comprise at least one of a first electrode, a second electrode, and a third electrode in the housing surrounding at least a part of the screen, wherein the first electrode is disposed on a left end of each of a plurality of first side surfaces along a long axis with respect to the front surface of the housing to be in contact with a left upper body of the subject, the second electrode is disposed on a right end of each of the plurality of first side surfaces along the long axis with respect to the front surface of the housing to be in contact with a right upper body of the subject, and the third electrode is disposed on a rear surface of the housing to be in contact with a lower body of the subject.

4. The mobile terminal of claim 1, wherein the one or more electrodes comprise at least one of a first electrode, a second electrode, and a third electrode in the housing surrounding at least a part of the screen, wherein the first electrode is disposed on a left edge of one of an upper end and a lower end of the housing to be in contact with a left upper body of the subject, the second electrode is disposed on a right edge of the other of the upper end and the lower end of the housing to be in contact with a right upper body of the subject, and the third electrode is disposed on one of a plurality of first side surfaces of the housing along a long axis to be in contact with an upper body of the subject.

5. The mobile terminal of claim 1, wherein the one or more electrodes comprise at least one of a first electrode, a second electrode, and a third electrode in the housing surrounding at least a part of the screen, wherein the first electrode is disposed on one of a plurality of first side surfaces of the housing along a long axis to be in contact with a hand of the subject gripping the housing, the second electrode is disposed on one of the plurality of first side surfaces of the housing along the long axis and a plurality of second side surfaces of the housing along a short axis to be in contact with another hand of the subject, and the third electrode is disposed on the other of the plurality of second side surfaces of the housing along the short axis to be in contact with an upper body of the subject.

6. The mobile terminal of claim 1, wherein the one or more electrodes comprise at least one of a first electrode, a second electrode, and a third electrode in the housing surrounding at least a part of the screen, wherein the first electrode is disposed on one of a plurality of first side surfaces of the housing along a long axis to be in contact with a left upper body of the subject, the second electrode is disposed on one of second side surfaces of the housing along a short axis to be in contact with a right upper body of the subject, and the third electrode is disposed on the other of the second side surfaces of the housing along the short axis to be in contact with an upper body of the subject.

7. The mobile terminal of claim 1, wherein the one or more electrodes comprise at least one of a first electrode, a second electrode, and a third electrode in the housing surrounding at least a part of the screen, wherein the first electrode is disposed on one of a plurality of second side surfaces of the housing along a short axis to be in contact with a left upper body of the subject, the second electrode is disposed on the other of the plurality of second side surfaces of the housing along the short axis to be in contact with a right upper body of the subject, and the third electrode is disposed on at least one of a plurality of first side surfaces of the housing along a long axis to be in contact with an upper body of the subject.

8. The mobile terminal of claim 1, wherein the one or more processors are configured to determine whether the subject complies with the usage guidelines and further output a notification signal based on the compliance with the usage guidelines.

9. The mobile terminal of claim 1, wherein the one or more processors are configured to further output a physical examination result of the subject by using a pre-learned model when input information comprising biosignals of the subject is input.

10. The mobile terminal of claim 9, wherein the input information comprises at least one of and clinical information, baseline electrocardiogram information, and biosignals of the subject comprising 6-lead electrocardiogram (I, II, III, aVR, aVL, aVF), and the physical examination result of the subject comprises at least one of a trend graph, a heart rhythm, a disease diagnosis, a heart function evaluation, and a physical condition evaluation.

11. The mobile terminal of claim 1, wherein the mobile terminal comprises a smartphone.

12. A method of measuring biosignals performed by a mobile terminal, the method comprising:
receiving, through one or more electrodes disposed on the mobile terminal,
biosignals of a subject in contact with the one or more electrodes; and
outputting at least one of a biosignal inspection result and usage guidelines regarding the electrode contact of the subject to a screen of the mobile terminal.

13. The method of claim 12, wherein the method of measuring biosignals comprises
determining whether the subject complies with the usage guidelines, and
outputting a notification signal based on the compliance with the usage guidelines.

14. The method of claim 12, wherein the outputting of the notification signal comprises outputting a physical examination result of the subject by using a pre-learned model when the terminal receives input information comprising the biosignals of the subject.

15. A system for measuring biosignals, the system comprising:
a mobile terminal for measuring biosignals according to claim 1;
a sensor for sensing biosignals of a subject by using one or more electrodes in the housing of a device for measuring biosignals; and
a server for obtaining, learning, and analyzing the biosignals of the subject, and providing an analysis result to the device for measuring the biosignals.

16. The system of claim 15, wherein the one or more electrodes comprise at least one of a first electrode, a second electrode, and a third electrode in the housing surrounding at least a part of the screen, wherein the first electrode is disposed on a left short end of at least one of a front surface and a rear surface of the housing to be in contact with a left upper body of the subject, the second electrode is disposed on a right short end of at least one of the front surface and the rear surface of the housing to be in contact with a right upper body of the subject, and the third electrode is disposed on the rear surface of the housing to be in contact with a lower body of the subject.

17. The system of claim 15, wherein the one or more electrodes comprise at least one of a first electrode, a second electrode, and a third electrode in the housing surrounding at least a part of the screen, wherein the first electrode is disposed on a left end of each of a plurality of first side surfaces along a long axis with respect to a front surface of the housing to be in contact with a left upper body of the subject, the second electrode is disposed on a right end of each of the plurality of first side surfaces along the long axis with respect to the front surface of the housing to be in contact with a right upper body of the subject, the third electrode is disposed on a rear surface of the housing to be in contact with a lower body of the subject.

18. The system of claim 15, wherein the one or more electrodes comprise at least one of a first electrode, a second electrode, and a third electrode in the housing surrounding at least a part of the screen, wherein the first electrode is disposed on a left edge of one of an upper end and a lower end of the housing to be in contact with a left upper body of the subject, the second electrode is disposed on a right edge of the other of the upper end and the lower end of the housing to be in contact with a right upper body of the subject, and the third electrode is disposed on one of a plurality of first side surfaces of the housing along a long axis to be in contact with an upper body of the subject

19. The system of claim 15, wherein the one or more electrodes comprise at least one of a first electrode, a second electrode, and a third electrode in the housing surrounding at least a part of the screen, wherein the one or more electrodes comprise at least one of a first electrode, a second electrode, and a third electrode in the housing surrounding at least a part of the screen, wherein the first electrode is disposed on one of a plurality of first side surfaces of the housing along a long axis to be in contact with a hand of a subject gripping the housing, the second electrode is disposed on one of the plurality of first side surfaces of the housing along the long axis and a plurality of second side surfaces of the housing along a short axis to be in contact with another hand of the subject, and the third electrode is disposed on the other of the plurality of second side surfaces of the housing along the short axis to be in contact with an upper body of the subject.

20. The system of claim 15, wherein the one or more electrodes comprise at least one of a first electrode, a second electrode, and a third electrode in the housing surrounding at least a part of the screen, wherein the first electrode is disposed on one of a plurality of first side surfaces of the housing along a long axis to be in contact with a left upper body of the subject, the second electrode is disposed on one of second side surfaces of the housing along a short axis to be in contact with a right upper body of the subject, and the third electrode is disposed on the other of the second side surfaces of the housing along the short axis to be in contact with an upper body of the subject.

21. The system of claim 15, wherein the one or more electrodes comprise at least one of a first electrode, a second electrode, and a third electrode in the housing surrounding at least a part of the screen, wherein the first electrode is disposed on one of a plurality of second side surfaces of the housing along a short axis to be in contact with a left upper body of the subject, the second electrode is disposed on the other of the plurality of second side surfaces of the housing along the short axis to be in contact with a right upper body of the subject, and the third electrode is disposed on at least one of a plurality of first side surfaces of the housing along a long axis to be in contact with an upper body of the subject.

22. The system of claim 15, wherein the device for measuring biosignals is configured to determine whether the subject complies with the usage guidelines, and further output a notification signal based on the compliance with the usage guidelines.

23. The system of claim 15, wherein the device for measuring biosignals is configured to further output a physical examination result of the subject using a pre-learned model upon inputting input information comprising the biosignals of the subject

24. The system of claim 23, wherein the input information comprises at least one of clinical information, baseline electrocardiogram information, and biosignals of the subject comprising 6-lead electrocardiogram (I, II, III, aVR, aVL, aVF), and the physical examination result of the subject comprises at least one of a trend graph, a heart rhythm, a disease diagnosis, a heart function evaluation, and a physical condition evaluation.
